# EUROPEAN PATENT APPLICATION

(11) **EP 4 575 618 A1**
(43) Date of publication of application: **25.06.2025**
(21) Application number: 23854435.7
(22) Date of filing: 15.08.2023
(51) Int. Cl.: G02C 13/00, A61L 12/10, A61L 101/06

(54) **AUTOMATIC CONTACT LENS CARE DEVICE**

(30) Priority: 17.08.2022 CN 202210988484
(71) Applicant: Suzhou 3N Biological Technology Co., Ltd, Suzhou, Jiangsu 215000 (CN)
(72) Inventor: SUN, Bixia, Suzhou, Jiangsu 215000 (CN); SHEN, Haipeng, Suzhou, Jiangsu 215000 (CN); DING, Mengyao, Suzhou, Jiangsu 215000 (CN); XU, Shengjiang, Suzhou, Jiangsu 215000 (CN); JIA, Lei, Suzhou, Jiangsu 215000 (CN); SUN, Ruichun, Suzhou, Jiangsu 215000 (CN); WANG, Yuan, Suzhou, Jiangsu 215000 (CN)
(74) Representative: Gille Hrabal Partnerschaftsgesellschaft mbB Patentanwälte
(86) International application number: PCT/CN2023/113089
(87) International publication number: WO 2024/037534

(57) **Abstract**

The present invention discloses an automatic contact lens care device, including a cover body, a base body, a washing assembly, a driving mechanism, and at least one storage device. The base body has an inner cavity and an opening communicating with the inner cavity. The cover body is arranged on the opening side of the base body. The washing assembly includes a first brush head and a second brush head. The first brush head is arranged on a side of the cover body facing the base body. The second brush head is arranged in the inner cavity. The first brush head and the second brush head are made of a soft material. The first brush head and the second brush head are correspondingly arranged in a matched manner. A contact lens can be placed between the first brush head and the second brush head. The first brush head and the second brush head can be driven by the driving mechanism to rotate relative to each other. The storage device communicates with the inner cavity. According to this application, the washing assembly is arranged in a cleaning chamber, and an electrophoresis dissociation technology and a physical rubbing technology can be combined, thereby greatly improving the cleaning effect; and the storage device is further arranged to automatically fill the liquid into the cleaning chamber or discharge the liquid from the cleaning chamber, thereby increasing the usage convenience and improving the user experience.

## Description

### Field of the Invention

The present invention relates to the technical field of contact lens cleaning devices, and in particular to, an automatic hard contact lens care device.

### Background technology

Contact lenses include soft contact lenses and hard contact lenses. Hard contact lenses further include hard corneal contact lenses, scleral lenses, etc.

How to clean hard corneal contact lenses and scleral lenses has always been a difficult problem in the industry. Taking a hard corneal contact lens as an example, during daily use, there may be residual deposits such as proteins and lipids, or bacteria on the hard corneal contact lens. However, the material structure of the hard corneal contact lens has a large number of oxygen permeable holes that are not visible to naked eyes, and the deposits such as proteins and lipids can easily permeate into the oxygen permeable holes, thereby causing a decrease in the oxygen permeability of the lens, and further causing symptoms such as corneal hypoxia and edema. In severe cases, problems such as corneal damage, bacterial infection, keratitis, and even visual impairment may be caused. Furthermore, during daily use, the contact lens may also have grease dirt, bacteria, and the like on the surface, which may affect the health of the contact lens.

In order to remove protein deposits on the surface of the contact lens, a traditional cleaning method is to physically rub the lens manually with a care solution, or soak the lens with a chemical active agent to achieve the purpose of removing proteins. However, both domestic and international experimental data and user feedback have shown that these methods have a little effect on removing proteins. Moreover, the process of finger rubbing and especially rubbing of the hard corneal contact lens easily scratches and damages the lens. Furthermore, the cleaning manner of finger rubbing is too cumbersome, which is not conducive to the user experience.

In addition, during use, the contact lens may also be contaminated with and breed bacteria. How to conveniently remove the bacteria is also a relatively troublesome matter.

Therefore, the applicant creatively applies an electrophoresis dissociation technology to hard contact lens care. The electrophoresis, that is, an abbreviation for the phenomenon of electrophoresis, refers to a phenomenon in which charged particles move towards an electrode opposite to the electrical property thereof under the action of an electric field. A technology that achieves separation by means of different movement speeds of charged particles in the electric field is referred to as an electrophoretic technology. As a technology for analyzing proteins, serum protein electrophoresis (SPE) is used. Proteins carry negative charges or positive charges in a buffer solution, and move to a positive electrode or a negative electrode in the electric field, so as to be referred to as electrophoresis. Different protein molecules have different electrophoretic mobilities. Electrolysis is a process in which a current passes through an electrolyte solution or an electrolyte (also referred to as an electrolytic solution) in a molten state to cause redox reactions on the negative electrode and the positive electrode. When a direct current voltage is applied to an electrochemical battery, an electrolytic process may occur. The applicant can achieve the technical effects of protein removal and sterilization to a certain extent by means of electrophoresis and electrolysis.

Although the electrophoresis dissociation technology can already remove proteins, bacteria, and the like on the hard corneal contact lens, some lipids or relatively large particle deposits such as gum in the eyes need to be rubbed by hand first, or need to be removed by increasing the current or prolonging the cleaning time. However, both increasing the current and prolonging the cleaning time may lead to an excessively strong oxidizing environment, and the excessively strong oxidizing environment may lead to deformation of the material of the hard contact lens or cause other problems affecting safe use.

In addition, an existing contact lens care device uses an operating manner of manually adding a liquid and pouring the liquid. Such a manner not only has cumbersome operations and requires a user to add and replace a cleaning liquid many times, but also has a case that the liquid splashes or the liquid is added outside a cleaning chamber.

Therefore, in the prior art, it is very difficult to well remove relatively large lipid or particle deposits or grease from the hard contact lens while not damaging the hard contact lens. In combination with the foregoing technical problems, new innovations are necessary.

### SUMMARY OF THE INVENTION

An objective of the present invention is to provide an automatic contact lens care device. A unique washing assembly is additionally arranged in a cleaning chamber, and an electrophoresis dissociation technology and a physical rubbing technology are combined, thereby greatly improving the cleaning effect of a hard contact lens. In addition, a storage device is further arranged and is configured to store a care solution, normal saline, clear water, waste liquid, or the like, so as to automatically fill the liquid into the cleaning chamber or discharge the liquid from the cleaning chamber, thereby increasing the usage convenience and improving the user experience.

In order to achieve the objective of the present invention, the present invention provides an automatic contact lens care device, including a cover body, a base body, a washing assembly, a driving mechanism, and at least one storage device; the base body has an inner cavity, a side of the base body has an opening, the opening communicates with the inner cavity, and the cover body is arranged on the opening side of the base body; the washing assembly includes a first brush head and a second brush head, the first brush head is arranged on a side of the cover body facing the base body, the second brush head is arranged in the inner cavity, and the first brush head and the second brush head are made of a soft material; the first brush head and the second brush head of the washing assembly are correspondingly arranged in a matched manner, a contact lens is able to be placed between the first brush head and the second brush head, and the first brush head and the second brush head are able to be driven by the driving mechanism to rotate relative to each other; and the storage device communicates with the inner cavity of the base body.

Further, the first brush head is configured as a brush head presenting a shape of an arc-shaped groove, and the second brush head is configured as a brush head presenting a shape of an arc-shaped protrusion; and/or the first brush head is configured as a brush head presenting a shape of an arc-shaped protrusion, and the second brush head is configured as a brush head presenting a shape of an arc-shaped groove.

Further, a side of the first brush head facing the second brush head has a flexible groove structure, and a side of the second brush head facing the first brush head has a flexible protrusion structure.

Further, a side of the first brush head facing the second brush head is provided with a plurality of first flexible structures, a side of the second brush head facing the first brush head is provided with a plurality of second flexible structures, the heights of the plurality of first flexible structures increase progressively from a middle position of the first brush head to the periphery to form a groove shape, and the heights of the plurality of second flexible structures decrease progressively from a middle position of the second brush head to the periphery to form a protrusion shape.

Further, a side of the second brush head facing the first brush head is provided with a convex limiting structure, the contact lens is able to be placed in a space defined by the second brush head, the limiting structure thereof and the first brush head, and the limiting structure is able to limit the contact lens from sliding out of the second brush head.

Further, the limiting structure is made of a soft material, and the limiting structure is a plurality of convex flexible posts or convex flexible barrier walls.

Further, a suction device is arranged between the storage device and the base body, and the suction device is configured to convey a substance in the storage device into the inner cavity of the base body; and/or the suction device is configured to convey a substance in the inner cavity of the base body into the storage device.

Further, the storage device includes a first storage device and a second storage device, a first suction device is arranged between the first storage device and the base body, a second suction device is arranged between the second storage device and the base body, the first suction device is configured to be able to convey a substance in the first storage device into the inner cavity of the base body, and the second suction device is configured to be able to convey a substance in the inner cavity of the base body into the second storage device.

Further, at least one first electrode component and at least one second electrode component are further arranged in the inner cavity; and after being powered on, the first electrode component and the second electrode component have opposite polarities.

Further, an electrolyte solution is able to be placed in the inner cavity, and the electrolyte solution is a solution containing chloride ions; the contact lens to be cleaned is able to be placed in the inner cavity filled with the solution containing chloride ions, the first electrode component and the second electrode component form a positive electrode and a negative electrode under a circuit loop, dacryolin attached to a surface of the contact lens to be cleaned is charged in the solution containing chloride ions, and the charged dacryolin moves towards an electrode position opposite to an electrical property thereof; and chloride ions in the electrolyte solution move towards the positive electrode and lose electrons to be oxidized into chlorine gas, and the chlorine gas is dissolved in the electrolyte solution to generate hypochlorous acid.

Further, the driving mechanism includes a first driving device, the second brush head is rotationally connected to the base body, and the first driving device is configured to be able to drive the second brush head to rotate.

Further, the driving mechanism includes a second driving device, the first brush head is rotationally connected to the cover body, and the second driving device is configured to be able to drive the first brush head to rotate.

Further, the driving mechanism includes a third driving device, and the third driving device is configured to be able to drive the cover body to be close to or away from the base body.

Further, the automatic contact lens care device further includes a housing and a pull component, where the housing has an accommodating cavity inside, a through port is arranged on one side of the housing, the through port communicates with the accommodating cavity, the pull component and the cover body are separately and movably arranged in the accommodating cavity, the base body is arranged on the pull component, and the pull component is able to be driven to move the base body out of the housing from the through port.

Further, the driving mechanism includes a fourth driving device, and the fourth driving device is configured to be able to drive the pull component to move.

Further, an elastic component is further arranged between the base body and the pull component.

Further, the automatic contact lens care device further includes a vibration component, and the vibration component is configured to be able to drive the base body to vibrate.

Further, the base body and the pull component are fastened through a fastener.

Compared with the prior art, the automatic contact lens care device of this application at least has one or more beneficial effects as follows:

In the automatic contact lens care device of this patent application, the flexible washing assembly is arranged in the cleaning chamber, and a hard contact lens, such as a hard corneal contact lens or a scleral lens, can be physically rubbed during a cleaning process. A flexible washing assembly simulates a brush head having a similar shape of an adult finger to physically rub a lens surface through rotation. Another flexible washing assembly is configured as a flexible structure which is made of a flexible material and is similar to toothbrush hair, where the first brush head and the second brush head respectively rub two sides of the lens through rotation, and it may be that the first brush head and the second brush head both rotate in opposite directions, or one rotates and the other does not move, so as to effectively remove dirt such as lipids or relatively large particle deposits such as gum in the eyes on the hard contact lens without increasing the current or prolonging the cleaning time to meet the cleaning requirement, thereby meeting the cleaning requirement without damaging the hard corneal contact lens and also without manual hand rubbing. The entire cleaning and care time can be controlled within several minutes.

The automatic contact lens care device described in this patent is provided with two brush heads of a washing assembly. A flexible brush head structure for rubbing a hard corneal contact lens or a scleral lens may be made of a soft material such as silicone. In this way, when a hard contact lens is rubbed, the lens is well protected. The height of the flexible structure at the top of the brush head below is designed, so that the top of the brush head has a convex shape. The height of the flexible structure at the bottom of the brush head above is designed, so that the bottom of the brush head has a concave shape. In this way, when removing the hard corneal contact lens, a user may directly place the hard corneal contact lens on the flexible structure of the lower brush head without reversing the direction of the hard corneal contact lens. Similarly, when the user wears the cleaned hard corneal contact lens, the hard corneal contact lens does not need to be turned and can be directly worn. Thus, the operation is convenient, and the user experience can be greatly improved. Furthermore, a circle of limiting structure may be further arranged around the flexible structure at an upper end of the lower brush head to achieve a limiting effect, thereby ensuring the stability of a lens placement position, and preventing the lens from sliding out of a specific station when the first brush head and the second brush head operate. The limiting structure may be a circle of limiting barrier walls or a circle of limiting posts.

The automatic contact lens care device described in this patent is further provided with a plurality of storage devices configured to store a care solution, normal saline, clear water or dirty water, so as to automatically fill the liquid into the cleaning chamber or discharge the liquid from the cleaning chamber, thereby increasing the usage convenience and improving the user experience. The storage device may be connected to the cleaning chamber in various manners. For example, a connection manner in which a pipeline cooperates with a valve may be used. The storage device configured to store the care solution or clear water is arranged at a relatively high position, and the storage device configured to store the dirty water is arranged at a relatively low position. Under the action of gravity, automatic liquid adding or discharge can also be implemented in the cleaning chamber by adjusting the opening or closing of the valve. A connection manner in which a suction device cooperates with a pipeline may also be used. The care solution or clear water in the storage device is sucked into the cleaning chamber through the suction device, or the dirty water in the cleaning chamber is sucked into the storage device for containing dirty water. Certainly, the storage device configured to store the care solution or clear water and the storage device configured to store the dirty water may also be placed in parallel. The clean solution is placed into the cleaning chamber through a water suction device, and the sewage is sucked into the storage device for containing dirty water through the water suction device.

The automatic contact lens care device described in this patent is further provided with a pull component to facilitate a user to remove and place a hard corneal contact lens. The pull component may also be driven by a driving device to achieve automatic pull, which improves the degree of automation of the automatic contact lens care device, is more convenient for a user to use, and achieves better user experience.

The automatic contact lens care device described in this patent is provided with a vibration device. After the care device is used for a period of time, vibration may be provided by the vibration device in cooperation with high-speed rotation of the brush head to clean the inside of the cleaning chamber, thereby achieving the self-cleaning of the device. The inside of the cavity may be self-cleaned, and residues on the lens may be further removed.

The elastic component is arranged between the base body and the pull component of the automatic contact lens care device described in this patent to avoid the rigid contact between the base body and the pull component. On the one hand, when the cover body is clamped with the base body, a buffering effect is achieved to prevent an excessively large clamping force between the upper brush head and the lower brush head from causing damage to the lens. On the other hand, a part of vibration generated by the vibration device may also be counteracted to prevent the entire housing from vibrating when the vibration device drives the base body to vibrate. A bayonet is used for clamping the base body and the pull component, so that the base body is more convenient to disassemble and assemble.

The automatic washing mechanism of the present invention may be combined with the electrophoresis dissociation technology and in combination with an electrolyte solution containing chloride ions, which not only can effectively separate pollutants such as dacryolin from the hard corneal contact lens, but also can generate hypochlorite and hypochlorous acid in the cleaning chamber, and not only can decompose stubborn sedimentary proteins on the hard corneal contact lens into small molecule proteins and amino acids to make electrophoretic absorption easier, but also can kill bacteria and pathogenic microorganisms to achieve double effects of protein removal and sterilization and disinfection. The used electrolyte solution may be any solution which does not contain heavy metals and contains chloride ions, such as normal saline and ordinary care solutions, and has very strong applicability. Furthermore, the flexible automatic washing mechanism enables dirt such as grease to be removed more easily, which is more beneficial to long-term effective care of the hard contact lens, and greatly improves the user experience.

### BRIEF DESCRIPTION OF THE DRAWINGS

FIG. 1 is a schematic structural diagram of a cover body and a base body provided in an embodiment of this application;
FIG. 2 is a schematic structural diagram of a base body provided in an embodiment of this application;
FIG. 3 is a half-sectional schematic structural diagram when a base body and a cover body provided in an embodiment of this application are clamped;
FIG. 4 is a schematic diagram of a position state between two brush heads in a washing assembly provided in an embodiment of this application;
FIG. 5 is a three-dimensional schematic structural diagram of an automatic contact lens care device with a housing provided in an embodiment of this application;
FIG. 6 and FIG. 7 are respectively three-dimensional schematic structural diagrams of the automatic contact lens care device shown in FIG. 5 with the housing and a storage device removed;
FIG. 8 is a schematic structural diagram of a pull component provided with a base body provided in an embodiment of this application;
FIG. 9 is a half-sectional schematic structural diagram of the pull component shown in FIG. 8 at the position of the base body; and
FIG. 10 and FIG. 11 are respectively half-sectional schematic structural diagrams of the automatic contact lens care device shown in FIG. 5.

In the figures, 10-cover body, 11-upper cover of cover body, 12-lower shell of cover body, 13-second accommodating cavity, 20-base body, 21-upper shell of base body, 22-lower shell of base body, 23-inner cavity, 24-first accommodating cavity, 25-drainage port, 30-washing assembly, 31-first brush head, 311-first flexible structure, 312-first rotating shaft, 313-first driven gear, 32-second brush head, 321-second flexible structure, 322-limiting structure, 323-second rotating shaft, 324-second driven gear, 40-storage device, 41-first storage device, 42-second storage device, 50-first electrode component, 60-second electrode component, 70-suction device, 71-first suction device, 72-second suction device, 80-first driving device, 81-first drive gear, 90-second driving device, 91-second drive gear, 92-fixed support, 100-third driving device, 101-guide shaft, 110-housing, 111-through port, 112-upper supporting seat, 1121-accommodating hole, 113-lower supporting seat, 1131-sliding groove, 114-fixed shell, 120-pull component, 121-groove structure, 122-convex strip, 123-rack structure, 130-fourth driving device, 131-driving gear, 140-elastic component, 150-fastener, 160-supporting seat, 170-first conductive element, and 180-contact lens.

### DETAILED DESCRIPTION OF THE PREFERRED EMBODIMENTS

To further explain the technical means used in the present invention for achieving the intended objectives and the effects thereof, the following describes specific implementations, structures, features and effects of the present invention in detail with reference to the accompanying drawings and preferred embodiments.

### Embodiments

This embodiment provides an automatic contact lens care device which is mainly composed of a cover body 10, a base body 20, a washing assembly 30, a storage device 40, and a driving mechanism.

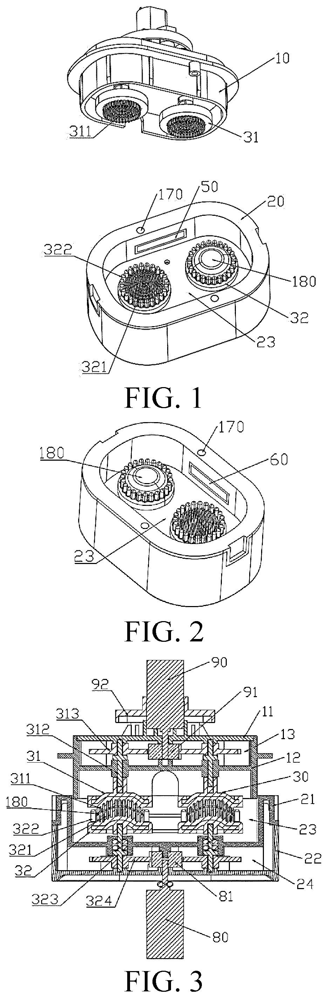

As shown in FIG. 1 to FIG. 3, the base body 20 has an inner cavity 23, that is, a cleaning chamber. A side, that is, an upper side, of the base body 20 has an opening. The opening communicates with the inner cavity 23. The cover body 10 is arranged on the opening side of the base body 20, that is, located above the base body 20. A total of two washing assemblies 30 are schematically shown in the figures. However, during a specific implementation, the number of the washing assembly 30 is not limited, and may be any number.

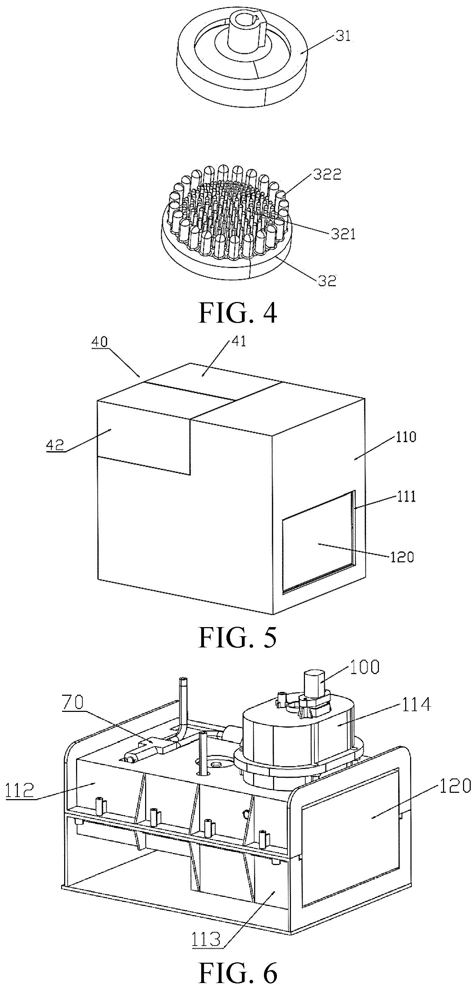

Each of the washing assemblies 30 is mainly composed of a first brush head 31 and a second brush head 32, as shown in FIG. 4. The first brush head 31 and the second brush head 32 are made of a soft material. The first brush head 31 is arranged on a side of the cover body 10 facing the base body 20. The second brush head 32 is arranged in the inner cavity 23. The first brush head 31 and the second brush head 32 of the washing assembly 30 are correspondingly arranged in a matched manner. The first brush head 31 and the second brush head 32 can be driven by the driving mechanism to rotate relative to each other. As shown in FIG. 3, the first brush head 31 and the second brush head 32 that are schematically shown in the figure are both arranged rotationally. The base body 20 is composed of an upper shell 21 of the base body and a lower shell 22 of the base body. The upper shell 21 of the base body is clamped above the lower shell 22 of the base body. The inner cavity 23 is arranged on the upper shell 21 of the base body. A first accommodating cavity 24 is formed between the upper shell 21 of the base body and the lower shell 22 of the base body. The second brush head 32 is rotationally connected to the upper shell 21 of the base body through a second rotating shaft 323. One end of the second rotating shaft 323 extends into the first accommodating cavity 24. The second rotating shaft 323 in the first accommodating cavity 24 is sheathed in a second driven gear 324. The driving mechanism includes a first driving device 80 such as a gear motor. A main shaft of the first driving device 80 is sheathed in a first drive gear 81. The first drive gear 81 is meshed with the second driven gear 324, so that under gear transmission, the first driving device 80 drives the second brush head 32 to rotate.

The first brush head 31 and the second brush head 32 may also be made of a flexible silicone material into protrusions and grooves, and do not have a plurality of brush head components such as toothbrush hair. A brush head having a flexible protrusion structure simulates a human finger, and another brush head having a flexible groove structure simulates a human palm, so that the assembly simulates human hand rubbing during operation. An optional embodiment is that the surfaces of the brush head having the flexible protrusion structure and the brush head having the flexible groove structure are also provided with fingerprint-like micro-protrusion structures by simulating human fingers to facilitate better cleaning.

An embodiment of the washing assembly is that an upper brush head has a shape of a groove, and a lower brush head has a protrusion structure. In this way, during a cleaning process, a reflection arc area and an inner side area of the contact lens are not easy to pollute, and moreover, a process of removing the contact lens from human eyes or placing the contact lens on human eyes is also facilitated, thereby satisfying ergonomics.

The cover body 10 is composed of an upper cover 11 of the cover body and a lower shell 12 of the cover body. The upper cover 11 of the cover body is clamped on a side of the lower shell 12 of the cover body away from the base body 20. A second accommodating cavity 13 is formed between the upper cover 11 of the cover body and the lower shell 12 of the cover body. The first brush head 31 is rotationally connected to the lower shell 12 of the cover body through a first rotating shaft 312. One end of the first rotating shaft 312 extends into the second accommodating cavity 13. The first rotating shaft 312 in the second accommodating cavity 13 is sheathed in a first driven gear 313. The driving mechanism includes a second driving device 90 such as a gear motor. A main shaft of the second driving device 90 is sheathed in a second drive gear 91. The second drive gear 91 is meshed with the first driven gear 313, so that under gear transmission, the second driving device 90 drives the first brush head 31 to rotate. As shown in FIG. 3, preferably, a main body of the second driving device 90 is fixedly installed on the upper cover 11 of the cover body through a fixed support 92.

Certainly, the foregoing is merely a preferred manner. During a specific implementation, the first brush head 31 and the second brush head 32 may also be driven in other manners, and are not limited to a manner of motor-driven gear transmission. Moreover, in order to implement the relative rotation between the first brush head 31 and the second brush head 32, rotation directions of the first brush head 31 and the second brush head 32 may be opposite, or a rotation speed difference may exist between the first brush head and the second brush head. Certainly, during a specific implementation, only one of the first brush head 31 and the second brush head 32 may also be designed to be able to rotate. Similarly, the relative rotation between the two brush heads may also be implemented.

Preferably, the first brush head 31 is configured as a brush head presenting a shape of an arc-shaped groove, and the second brush head 32 is configured as a brush head presenting a shape of an arc-shaped protrusion. Further preferably, a side of the first brush head 31 facing the second brush head 32 has a flexible groove structure, and a side of the second brush head 32 facing the first brush head 31 has a flexible protrusion structure. For example, a side of the first brush head 31 facing the second brush head 32 is provided with a plurality of first flexible structures 311, and a side of the second brush head 32 facing the first brush head 31 is provided with a plurality of second flexible structures 321. The first flexible structure 311 and/or the second flexible structure 321 are/is preferably made of a soft material such as silicone. When the contact lens 180 is rubbed, a soft material such as silicone may be used for protecting the lens well. As shown in FIG. 3, the first brush head 31 and the second brush head 32 that are schematically shown in the figure are both composed of a main body of the brush head and a silicone sleeve which is sleeved over the main body of the brush head. The first flexible structure 311 or the second flexible structure 321 is a cylindrical structure extending out of the silicone sleeve. The heights of the plurality of first flexible structures 311 increase progressively from a middle position of the first brush head 31 to the periphery to form a groove shape; and the heights of the plurality of second flexible structures 321 decrease progressively from a middle position of the second brush head 32 to the periphery to form a protrusion shape, as shown in FIG. 1 to FIG. 4. In this way, when removing the contact lens 180, a user can directly place the contact lens on the flexible structure of the lower brush head without reversing the direction of the contact lens 180. Similarly, when the user wears the cleaned contact lens 180, the contact lens does not need to be turned and can be directly worn. Thus, the operation is convenient, and the user experience can be greatly improved. Furthermore, the sewage on the contact lens 180 will automatically flow away and will not form liquid residue on the contact lens 180. Further, a side of the second brush head 32 facing the first brush head 31 is further provided with a convex limiting structure 322, as shown in FIG. 4. The contact lens 180 can be placed in a space defined by the second brush head 32, the limiting structure 322 thereof and the first brush head 31. The limiting structure 322 can limit the contact lens 180 from sliding out of the second brush head 32. The limiting structure 322 is made of a soft material. For example, the limiting structure 322 may also be a plurality of convex flexible posts extending from the silicone sleeve. Compared with flexible structures, the limiting structure has higher strength, and can achieve a certain limiting effect to ensure the stability of a lens placement position. Certainly, the limiting structure 322 may also be other structures such as flexible barrier walls.

Certainly, the first brush head 31 may also be configured as a brush head presenting a shape of an arc-shaped protrusion, and the second brush head 32 may also be configured as a brush head presenting a shape of an arc-shaped groove. However, such an implementation may cause the formation of residual sewage on an inner side of the contact lens 180 after cleaning is completed. Moreover, the inner side of the contact lens 180 is provided with a reversing arc, and the reversing arc is relatively easy to dirty. Therefore, the inner side of the contact lens 180 is a cleaning focus, so the former manner of placing the contact lens 180 with the inner side downward is more preferable.

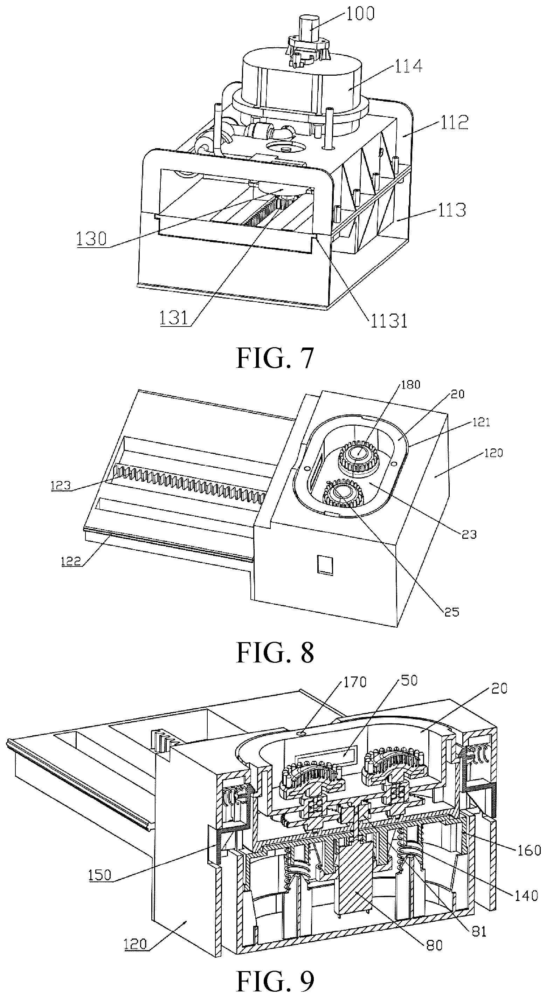

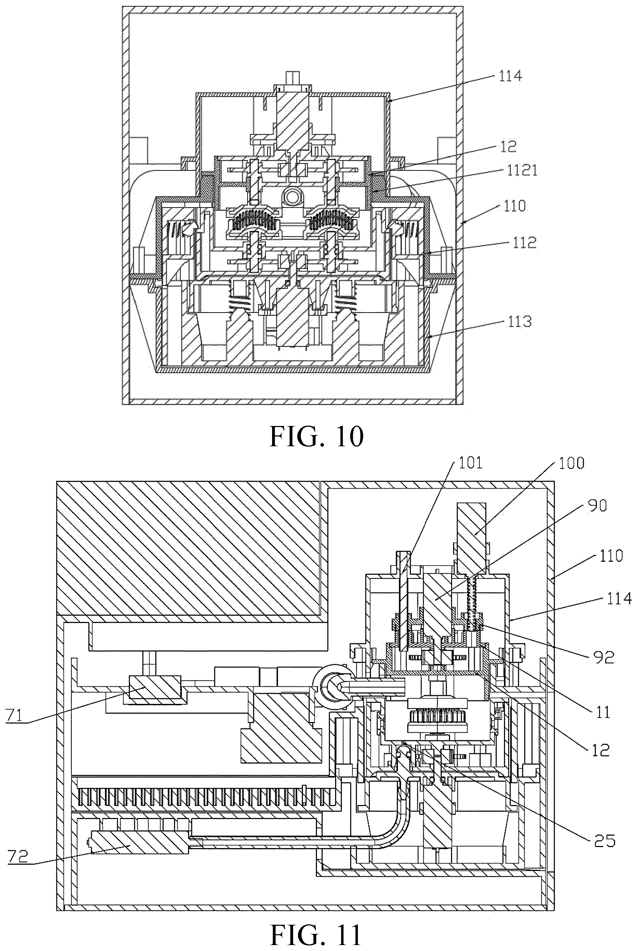

As shown in FIG. 5, two storage devices 40 are schematically shown, which are respectively defined as a first storage device 41 and a second storage device 42. Certainly, the number of the storage devices 40 is not limited to this, and may be any plurality, for example, one, three, or another plurality. Each storage device 40 separately communicates with the inner cavity 23 of the base body 20. Preferably, the storage device 40 communicates with the inner cavity 23 of the base body 20 through a pipeline. As shown in FIG. 1 and FIG. 3, schematically, a gap is formed on a side wall of a bottom end of the lower shell 12 of the cover body, and a pipeline on the first storage device 41 is inserted between the lower shell 12 of the cover body and the upper shell 21 of the base body through the gap, so as to communicate with the inner cavity 23 of the base body 20, as shown in FIG. 7, FIG. 10 and FIG. 11. A pipeline on the second storage device 42 communicates with a drainage port 25 located at the bottom of the inner cavity 23 on the upper shell 21 of the base body. Preferably, the pipeline of the storage device 40 is connected with a suction device 70 such as a water suction pump. For example, as shown in FIG. 6 and FIG. 11, the pipeline of the first storage device 41 is connected with a first suction device 71. A suction port of the first suction device 71 communicates with the inner cavity 23 of the first storage device 41 through the pipeline, and a discharge port communicates with the inner cavity 23 of the base body 20 through the pipeline. In this way, during a specific implementation, after a substance such as a care solution, normal saline, cleaning liquid or clear water is added to the first storage device 41, the first suction device 71 can automatically add the liquid to the inner cavity 23 of the base body 20. Similarly, the pipeline of the second storage device 42 is connected with a second suction device 72. A discharge port of the second suction device 72 communicates with the inner cavity 23 of the second storage device 42 through the pipeline, and a suction port communicates with the inner cavity 23 of the base body 20 through the pipeline. In this way, during a specific implementation, a substance such as dirty water or waste liquid in the inner cavity 23 of the base body 20 can be automatically discharged into the second storage device 42. Certainly, the communication manner of the storage device 40 and the inner cavity 23 of the base body 20 is not limited to this, and may also be another manner. For example, a connection manner in which a pipeline cooperates with a valve may be used. The storage device 40 configured to store normal saline, a care solution or clear water is arranged at a relatively high position, and the storage device 40 configured to store the dirty water is arranged at a relatively low position. Under the action of gravity, automatic liquid adding or discharge can also be implemented in the cleaning chamber by adjusting the opening or closing of the valve. It should be noted that when one storage device 40 is arranged, the storage device 40 not only may be configured to store a substance such as a care solution, normal saline, cleaning liquid or clear water, but also may be configured to store dirty water. A suction direction of the suction device 70 may be set according to needs so as to convey the substance in the storage device 40 into the inner cavity 23 of the base body 20, and/or convey the substance in the inner cavity 23 of the base body 20 into the storage device 40.

At least one first electrode component 50 and at least one second electrode component 60 are further arranged in the inner cavity 23 of the base body 20. After being powered on, the first electrode component 50 and the second electrode component 60 have opposite polarities. As shown in FIG. 1 and FIG. 2, schematically, the first electrode component 50 and the second electrode component 60 both have a sheet-like conductive plate structure, and are respectively arranged on two side walls of the inner cavity 23 on the upper shell 21 of the base body, where one is used as a positive electrode, and the other is used as a negative electrode. In this way, during operation of the care device, an electrolyte solution, such as a care solution, containing chloride ions is added to the inner cavity 23 of the base body 20. After the contact lens 180 to be cleaned is placed in the inner cavity 23 filled with the solution containing chloride ions, the first electrode component 50 and the second electrode component 60 are respectively powered on. A positive electrode and a negative electrode are formed between the first electrode component 50 and the second electrode component 60 under a circuit loop formed by the electrolyte solution, and have a certain potential difference. Pollutants, such as dacryolin, attached to the surface of the contact lens 180 to be cleaned are charged in the solution containing chloride ions. Under the action of the potential difference, the pollutants, such as the charged dacryolin, move towards an electrode position opposite to the electrical property thereof so as to be separated from the contact lens 180, thereby cleaning the contact lens 180. Chloride ions in the solution containing sodium chloride move towards the positive electrode and lose electrons to be oxidized into chlorine gas, and the chlorine gas is dissolved in the electrolyte solution to generate hypochlorous acid. The hypochlorous acid will slowly undergo the self-redox reaction to decompose. During decomposition, each hypochlorous acid molecule absorbs electrons. The hypochlorous acid, as a strong oxidant, can absorb electrons of surface proteins of microbial cell walls, thereby oxidizing functional proteins on microbial surfaces. Microorganisms ultimately become inactive due to their inability to absorb nutrients, abnormal metabolism and cessation of division, thereby finally achieving the effects of sterilization and disinfection to achieve the purpose of sterilization. Furthermore, hydrogen ions, chloride ions and oxygen gas are dissociated. During the process of sterilization and virus killing, the hypochlorous acid can act on cell walls and virus envelopes. Furthermore, due to their small size and lack of charge, hypochlorous acid molecules may also permeate into bacterial (viral) bodies and undergo oxidation reactions with organic macromolecules such as bacterial (viral) proteins, nucleic acids and enzymes, thereby killing pathogenic microorganisms.

Further, the cover body 10 also adopts an automatic driving design, that is, the cover body can be driven to be close to or away from the base body 20 automatically. As shown in FIG. 5 to FIG. 7, a preferred solution is schematically shown. A housing 110 and a pull component 120 are arranged. An upper corner of the housing 110 is provided with a gap, and the first storage device 41 and the second storage device 42 are arranged in the gap, so that the appearance of the automatic contact lens care device in this embodiment is neater on the whole, for example, the automatic contact lens care device presents a cuboid shape shown in FIG. 5. The housing 110 has an accommodating cavity. A side, for example, a side in a length direction, of the housing 110 is provided with a through port 111. The through port 111 communicates with the accommodating cavity. The pull component 120 and the cover body 10 are separately and movably arranged in the accommodating cavity. Specifically, an upper supporting seat 112 and a lower supporting seat 113 are arranged in the housing 110, and the upper supporting seat 112 is clamped above the lower supporting seat 113, as shown in FIG. 6. A pull chamber is formed between the upper supporting seat 112 and the lower supporting seat 113, and the pull chamber communicates with the through port 111. The pull component 120 is movably arranged in the pull chamber. The base body 20 is arranged on the pull component 120. The pull component 120 can be driven to move the base body 20 out of the housing 110 from the through port 111. As shown in FIG. 8 and FIG. 9, schematically, a groove structure 121 is arranged at the top of the pull component 120 close to the through port 111. A supporting seat 160 is arranged in the groove structure 121. An elastic component 140, such as a spring, is arranged between the bottom of the supporting seat 160 and the groove bottom of the groove structure 121. As shown in FIG. 9, two elastic components are arranged. The base body 20 is located in the groove structure 121, and is placed above the supporting seat 160 in an abutted manner. Preferably, the base body 20 and the inner wall of the groove structure 121 are fastened through a fastener 150. Under the cooperation between the fastener 150 and the elastic component 140, the supporting seat 160 can tightly abut against the base body 20. For ease of extraction, unlocking holes may further be formed at two sides of the pull device corresponding to the fastener 150, so as to more conveniently unlock the fastener 150 and extract the base body 20 out of the groove structure 121. As shown in FIG. 9, schematically, the first driving device 80 is arranged on a side of the supporting seat 160 away from the base body 20. A main shaft of the first driving device 80 extends upward through the supporting seat 160. The first drive gear 81 is preset in the first accommodating cavity 24, and a through hole is formed at the bottom of the lower shell 22 of the base body corresponding to the first drive gear 81. In this way, when the base body 20 is placed in the groove structure 121, the main shaft of the first driving device 80 may be inserted into the first drive gear 81 through the through hole.

An accommodating hole 1121 is arranged at the upper end of the upper supporting seat 112 close to the through port 111, and the accommodating hole 1121 communicates with the pull chamber, as shown in FIG. 10. The cover body 10 is installed in the accommodating hole 1121. Convex edges are arranged on an outer circumferential wall of the lower shell 12 of the cover body. As shown in FIG. 3, the convex edges may be used for supporting to ensure that the cover body 10 does not fall into the pull chamber when being installed in the accommodating hole 1121, as shown in FIG. 10. A fixed shell 114 is clamped into the upper supporting seat 112 corresponding to the accommodating hole 1121. The driving mechanism includes a third driving device 100 for driving the cover body 10 to move up and down in the inner cavity of the fixed shell 114, so that the cover body 10 can be close to or away from the base body 20 automatically. As shown in FIG. 10 and FIG. 11, a driving manner is schematically shown. The third driving device 100 is arranged on the fixed shell 114. A main shaft of the third driving device 100 is designed into a shape of a lead screw, penetrates into the fixed shell 114 and is in threaded connection with the fixed support 92. In this way, when the third driving device 100 operates, the fixed support 92 can drive the cover body 10 to move up and down by driving the main shaft to rotate forward and backward. In order to ensure the stability of movement of the cover body 10, a guide shaft 101 may further be arranged in the inner cavity of the fixed shell 114. The guide shaft 101 is arranged vertically. One end of the guide shaft 101 is connected to the fixed shell 114. A guide hole is arranged at a position of the fixed support 92 corresponding to the guide shaft 101 and is sleeved over the guide shaft 101, thereby achieving the guide. Further, holes may also be punched in positions of the upper cover 11 of the cover body corresponding to the guide shaft 101 and the main shaft of the third driving device 100, so that there is enough space to accommodate the guide shaft 101 and the main shaft of the third driving device 100 when the cover body 10 is elevated.

As shown in FIG. 7 to FIG. 9, a movable structural design of the pull component 120 is schematically shown. That is, sliding grooves 1131 are respectively arranged on two side walls of the inner cavity of the lower supporting seat 113. The length direction of the sliding groove 1131 is consistent with the length direction of the housing 110. Convex strips 122 are respectively arranged at corresponding positions of the pull component 120. When the pull component 120 is arranged in the pull chamber, the convex strips 122 are located in the corresponding sliding grooves 1131. By applying an acting force in the length direction of the housing 110 to the pull component 120, the pull component 120 can move in the pull chamber. As shown in FIG. 8, schematically, a rack structure 123 is arranged at an upper end of the pull component 120. The length direction of the rack structure 123 is consistent with the length direction of the housing 110. The driving mechanism includes a fourth driving device 130, such as a gear motor, which is arranged on an upper inner wall of the upper supporting seat 112. A main shaft of the fourth driving device 130 is sheathed in a driving gear 131. The driving gear 131 is meshed with the rack structure 123, as shown in FIG. 7. Therefore, under the transmission of the driving gear 131, the fourth driving device 130 may drive the pull component 120 to move in the pull chamber along the length direction of the housing 110.

Preferably, a first conductive element 170, such as a conductive probe, is arranged at an upper end of the base body 20 corresponding to each of the first electrode component 50 and the second electrode component 60. Another matched second conductive element (not shown in the figure) is respectively arranged at a corresponding position on the cover body 10. A circuit board configured to supply power to the first electrode component 50 and the second electrode component 60 may be arranged outside the base body 20, for example, inside the fixed shell 114. The second conductive element is connected to the circuit board. In this way, after the fourth driving device 130 drives the pull component 120 to enable the base body 20 to be located below the accommodating hole 1121, the third driving device 100 drives the cover body 10 to fall. When the first conductive element 170 abuts against and is connected to the corresponding second conductive element, the circuit board can supply power to the first electrode component 50 and the second electrode component 60.

Further, a vibration device may also be arranged, so that the base body 20 can vibrate. For example, a vibration motor and the like may be installed on the supporting seat 160. In this way, after the care device is used for a period of time, vibration may be provided by the vibration device in cooperation with high-speed rotation of the brush head to clean the inside of the cleaning chamber, thereby achieving the self-cleaning of the device.

In this specification, the terms "include," "contain," or any other variants thereof are intended to cover a non-exclusive inclusion, so that not only are those listed elements included but also other elements which are not expressly listed are included.

In this specification, the involved orientation words, such as "front," "rear," "above," and "below" are defined according to the positions of the components and the position relationship between the components in the accompanying drawings, and are merely used for clarity and convenience for expressing the technical solutions. It should be understood that the use of the orientation words should not limit the scope of protection of this application.

The foregoing descriptions are merely preferred embodiments of the present invention, and are not intended to limit the present invention. Any modifications, equivalent replacements, improvements, and the like made within the spirit and principle of the present invention shall be included within the scope of protection of the present invention.

## Claims

1. An automatic contact lens care device, comprising a cover body (10), a base body (20), a washing assembly (30), and a driving mechanism, wherein
the base body (20) has an inner cavity (23), a side of the base body (20) has an opening, the opening communicates with the inner cavity (23), and the cover body (10) is arranged on the opening side of the base body (20);
the washing assembly (30) comprises a first brush head (31) and a second brush head (32), the first brush head (31) is arranged on a side of the cover body (10) facing the base body (20), the second brush head (32) is arranged in the inner cavity (23), and the first brush head (31) and the second brush head (32) are made of a soft material; and
the first brush head (31) and the second brush head (32) of the washing assembly (30) are correspondingly arranged in a matched manner, a contact lens (180) is able to be placed between the first brush head (31) and the second brush head (32), and the first brush head (31) and/or the second brush head (32) are able to be driven by the driving mechanism to rotate relative to each other.

2. The automatic contact lens care device according to claim 1, wherein the first brush head (31) is configured as a brush head presenting a shape of an arc-shaped groove, and the second brush head (32) is configured as a brush head presenting a shape of an arc-shaped protrusion; and/or
the first brush head (31) is configured as a brush head presenting a shape of an arc-shaped protrusion, and the second brush head (32) is configured as a brush head presenting a shape of an arc-shaped groove.

3. The automatic contact lens care device according to claim 1, wherein a side of the first brush head (31) facing the second brush head (32) has a flexible groove structure, and a side of the second brush head (32) facing the first brush head (31) has a flexible protrusion structure.

4. The automatic contact lens care device according to claim 3, wherein the side of the first brush head (31) facing the second brush head (32) is provided with a plurality of first flexible structures (311), the side of the second brush head (32) facing the first brush head (31) is provided with a plurality of second flexible structures (321), heights of the plurality of first flexible structures (311) increase progressively from a middle position of the first brush head (31) to the periphery to form a groove shape, and heights of the plurality of second flexible structures (321) decrease progressively from a middle position of the second brush head (32) to the periphery to form a protrusion shape.

5. The automatic contact lens care device according to claim 4, wherein the side of the second brush head (32) facing the first brush head (31) is provided with a convex limiting structure (322), the contact lens (180) is able to be placed in a space defined by the second brush head (32), the limiting structure (322) thereof and the first brush head (31), and the limiting structure (322) is able to limit the contact lens (180) from sliding out of the second brush head (32).

6. The automatic contact lens care device according to claim 5, wherein the limiting structure (322) is made of a soft material, and the limiting structure (322) is a plurality of convex flexible posts or convex flexible barrier walls.

7. The automatic contact lens care device according to claim 1, further comprising:
at least one storage device (40) communicating with the inner cavity (23) of the base body (20);
a suction device (70) arranged between the storage device (40) and the base body (20), wherein the suction device (70) is configured to convey a substance in the storage device (40) into the inner cavity (23) of the base body (20); and/or
the suction device (70) is configured to convey a substance in the inner cavity (23) of the base body (20) into the storage device (40).

8. The automatic contact lens care device according to claim 1, further comprising:
at least one storage device (40) communicating with the inner cavity (23) of the base body (20); and
the storage device (40) comprises a first storage device (41) and a second storage device (42), a first suction device (71) is arranged between the first storage device (41) and the base body (20), a second suction device (72) is arranged between the second storage device (42) and the base body (20), the first suction device (71) is configured to be able to convey a substance in the first storage device (41) into the inner cavity (23) of the base body (20), and the second suction device (72) is configured to be able to convey a substance in the inner cavity (23) of the base body (20) into the second storage device (42).

9. The automatic contact lens care device according to claim 1, wherein at least one first electrode component (50) and at least one second electrode component (60) are further arranged in the inner cavity (23); and after being powered on, the first electrode component (50) and the second electrode component (60) have opposite polarities.

10. The automatic contact lens care device according to claim 9, wherein an electrolyte solution is able to be placed in the inner cavity (23), and the electrolyte solution is a solution containing sodium chloride;
the contact lens (180) to be cleaned is able to be placed in the inner cavity (23) filled with the solution containing sodium chloride, the first electrode component (50) and the second electrode component (60) form a positive electrode and a negative electrode under a circuit loop, dacryolin attached to a surface of the contact lens (180) to be cleaned is charged in the solution containing sodium chloride, and the charged dacryolin moves towards an electrode position opposite to an electrical property thereof; and chloride ions in the electrolyte solution move towards the positive electrode and lose electrons to be oxidized into chlorine gas, and the chlorine gas is dissolved in the electrolyte solution to generate hypochlorous acid.

11. The automatic contact lens care device according to claim 1, wherein the driving mechanism comprises a first driving device (80), the second brush head (32) is rotationally connected to the base body (20), and the first driving device (80) is configured to be able to drive the second brush head (32) to rotate.

12. The automatic contact lens care device according to claim 1, wherein the driving mechanism comprises a second driving device (90), the first brush head (31) is rotationally connected to the cover body (10), and the second driving device (90) is configured to be able to drive the first brush head (31) to rotate.

13. The automatic contact lens care device according to claim 1, wherein the driving mechanism comprises a third driving device (100), and the third driving device (100) is configured to be able to drive the cover body (10) to be close to or away from the base body (20).

14. The automatic contact lens care device according to claim 1, further comprising a housing (110) and a pull component (120), wherein the housing (110) has an accommodating cavity inside, a through port (111) is arranged on one side of the housing (110), the through port (111) communicates with the accommodating cavity, the pull component (120) and the cover body (10) are separately and movably arranged in the accommodating cavity, the base body (20) is arranged on the pull component (120), and the pull component (120) is able to be driven to move the base body (20) out of the housing (110) from the through port (111).

15. The automatic contact lens care device according to claim 14, wherein the driving mechanism comprises a fourth driving device (130), and the fourth driving device (130) is configured to be able to drive the pull component (120) to move.

16. The automatic contact lens care device according to claim 14, wherein an elastic component (140) is further arranged between the base body (20) and the pull component (120).

17. The automatic contact lens care device according to claim 16, further comprising a vibration component, wherein the vibration component is configured to be able to drive the base body (20) to vibrate.

18. The automatic contact lens care device according to claim 14, wherein the base body (20) and the pull component (120) are fastened through a fastener (150).
